(19) Europäisches Patentamt
European Patent Office
Office européen des brevets

(11) Veröffentlichungsnummer: **0 049 888**
**B1**

(12) # EUROPÄISCHE PATENTSCHRIFT

(45) Veröffentlichungstag der Patentschrift: **20.07.83**

(51) Int. Cl.³: **C 07 C 11/00,** C 07 C 1/02, B 01 J 23/84

(21) Anmeldenummer: **81108213.0**

(22) Anmeldetag: **12.10.81**

(54) **Verfahren zur Herstellung ungesättigter Kohlenwasserstoffe.**

(30) Priorität: **11.10.80 DE 3038450**

(43) Veröffentlichungstag der Anmeldung: **21.04.82 Patentblatt 82/16**

(45) Bekanntmachung des Hinweises auf die Patenterteilung: **20.07.83 Patentblatt 83/29**

(84) Benannte Vertragsstaaten: **DE FR GB IT NL**

(56) Entgegenhaltungen: **DE-A-2 518 964**

(73) Patentinhaber: **Ruhrchemie Aktiengesellschaft, Bruchstrasse 219, D-4200 Oberhausen 13 (DE)**

(72) Erfinder: **Cornils, Boy, Dr., Dipl.-Chem., Friedrich-Ebert-Strasse 45, D-4220 Dinslaken (DE)**
Erfinder: **Frohning, Carl Dieter, Dr. Dipl.-Chem., Elsenbruch 1, D-4200 Oberhausen 13 (DE)**
Erfinder: **Büssemeier, Bernd, Dr., Dipl.-Chem., Nikolaus-Ehlenstrasse 28, D-4330 Mülheim/Ruhr (DE)**

(74) Vertreter: **Reichelt, Karl-Heinz, Dr., m. Br. Ruhrchemie Aktiengesellschaft Abt. PLD Postfach 13 01 60, D-4200 Oberhausen 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 049 888 B1

# Verfahren zur Herstellung ungesättigter Kohlenwasserstoffe

Die vorliegende Erfindung betrifft die Herstellung von kurzkettigen Olefinen aus Synthesegas.

Bei der Hydrierung von Kohlenoxiden zu Olefinen ist der Umsatz in hohem Masse vom Wasserstoffpartialdruck abhängig. Je grösser der Wasserstoffpartialdruck ist, desto höhere Umsätze werden erzielt. Gleichzeitig nimmt jedoch mit steigendem Wasserstoffpartialdruck auch die Hydrierung der primär gebildeten Olefine zu. Daneben ist noch die Konvertierung des bei der Synthese gebildeten Reaktionswassers entsprechend

$$H_2O + CO \rightleftharpoons H_2 + CO_2$$

zu berücksichtigen, die unter Einwirkung der für die Kohlenoxidhydrierung verwendeten Katalysatoren abläuft. Dadurch geht ein nicht unerheblicher Anteil der eingesetzten Kohlenmonoxide verloren. In der Praxis stellt sich daher die Aufgabe, in Gegenwart selektiv wirkender Katalysatoren den Synthesedruck bzw. den Wasserstoffpartialdruck so einzustellen, dass die Kohlenoxidhydrierung mit hohem Umsatz abläuft und sowohl eine Hydrierung der primär gebildeten Olefine als auch die Konvertierung von Kohlenmonoxid mit Wasserdampf weitgehend unterbunden wird.

Die Herstellung ungesättigter, insbesondere gasförmiger Kohlenwasserstoffe durch Umsetzung von Kohlenoxid mit Wasserstoff in Gegenwart von Katalysatoren ist schon mehrfach beschrieben worden. So wird nach dem Verfahren der DT-PS 922 883 mit Eisenschmelzkatalysatoren gearbeitet, die periodisch oder kontinuierlich aus dem Reaktionsraum entfernt, regeneriert, reduziert und wieder zurückgeführt werden. Die Umsetzung wird bei gewöhnlichem oder leicht erhöhtem Druck und bei Temperaturen oberhalb etwa 450°C, zweckmässigerweise bei 470 bis 600°C durchgeführt.

Nach einer anderen, in der DE-PS 896 338 beschriebenen Arbeitsweise gewinnt man ungesättigte, gasförmige Kohlenwasserstoffe durch Umsetzung von Kohlenoxid mit Wasserstoff in Gegenwart beständiger Oxide der Metalle der II. bis VII. Gruppe des Periodensystems. Die Reaktion erfolgt bei etwa Atmosphärendruck und bei Temperaturen oberhalb 520°C.

Die bei den bekannten Verfahren angewandten hohen Reaktionstemperaturen haben entsprechend dem Boudouard-Gleichgewicht die Bildung von Kohlenstoff aus Kohlenmonoxid zur Folge. Die Kohlenstoffabscheidung führt zu einer Desaktivierung der Katalysatoroberfläche und im Einzelfall zu einer Sprengung des Katalysatorgefüges, wodurch die Lebensdauer des Katalysators beträchtlich herabgesetzt wird.

Alle konventionellen Kohlenoxidhydrierungs-Katalysatoren sind gegen Vergiftungen empfindlich. Besonders wirksame Gifte sind Schwefelverbindungen, die zur Bildung syntheseinaktiver Metallsulfide auf der Katalysatoroberfläche führen.

Man bemüht sich daher in zunehmendem Masse, schwefelresistente Katalysatoren für die Umsetzung von Kohlenoxid und Wasserstoff zu entwickeln, z.B. durch Einsatz von Molybdän, das wenig empfindlich gegen Schwefel ist, als Katalysatorkomponente (vgl. US-PS 41 51 190). Auf diesem Wege soll durch Senkung des bisher erforderlichen Aufwandes für die Gasreinigung die Wirtschaftlichkeit der Umsetzung erhöht werden.

Überraschenderweise wurde nun gefunden, dass durch eine gezielte Vergiftung mit Schwefel die Lebensdauer bestimmter Katalysatoren erhöht und ihre Aktivität und Selektivität verbessert werden können.

Die Erfindung besteht in einem Verfahren zur Herstellung ungesättigter niedermolekularer Kohlenwasserstoffe durch Hydrierung von Kohlenoxiden in Gegenwart von Katalysatoren, deren Hauptkomponenten Eisen und Vanadium sind und die daneben aus Magnesiumoxid und Kaliumoxid bestehen. Es ist dadurch gekennzeichnet, dass die Katalysatoren 100 Gewichtsteile Eisen, 50 bis 100 Gewichtsteile Vanadium, bis zu 10 Gewichtsteile Magnesiumoxid, 3 bis 5 Gewichtsteile Kaliumoxid und ausserdem 10 bis 150 ppm Schwefel (bezogen auf die gesamte Katalysatormasse) enthalten.

Es hat sich gezeigt, dass Katalysatoren der genannten Zusammensetzung eine um das Eineinhalb- bis Zweifache längere Lebensdauer besitzen und darüber hinaus zu einer um 40 bis 50% höheren Olefinausbeute führen, verglichen mit Katalysatoren, die keinen Schwefel enthalten.

Die Dotierung der Katalysatoren mit Schwefel kann auf verschiedenen Wegen erfolgen. So ist es möglich, den Schwefel in Form von Schwefelverbindungen wie Sulfiden oder Sulfaten in die Katalysatormasse direkt einzuführen oder den fertigen Katalysator mit gasförmigen Schwefelverbindungen wie Schwefelwasserstoff, Schwefelkohlenstoff oder Kohlenoxysulfid zu behandeln.

Besonders bewährt hat sich der Einsatz von schwefelhaltigem Magnesiumoxid als Katalysatorkomponente. Bevorzugt wird in diesem Falle Magnesiumoxid, das von der Herstellung oder durch Zumischen Sulfat enthält.

Die für das erfindungsgemässe Verfahren eingesetzten Katalysatoren enthalten bis zu 10 Gewichtsteile, vorzugsweise 2 bis 10 Gewichtsteile Magnesiumoxid (bezogen auf 100 Gewichtsteile Eisen).

Die Herstellung der Katalysatoren erfolgt z.B. durch Fällung der Bestandteile aus ihren wässrigen Lösungen mit geeigneten Fällungsreagenzien, wie Alkalimetallcarbonaten oder -hydroxiden. Ein anderes Verfahren besteht darin, die Bestandteile zu mischen, das Gemisch zu homogenisieren und auf mechanischem Wege zu verformen. Darüber hinaus können die Katalysatoren auch durch Sintern der pulverförmigen Bestandteile erhalten werden.

Unter dem Begriff Kohlenoxide werden Kohlenmonoxid und Kohlendioxid oder deren Gemische verstanden. Üblicherweise geht man zur Herstellung der Olefine nach dem erfindungsgemässen Verfahren von Synthesegas, d.h. dem Gemisch von Kohlenmonoxid und Wasserstoff aus, das aus minderwertigen Erdölfraktionen, insbesondere aber auch aus Kohle durch partielle Oxidation in Gegenwart von Wasserdampf nach bekannten Verfahren zugänglich ist.

Im Normalfall enthält Synthesegas etwa gleiche Volumenteile Kohlenmonoxid und Wasserstoff. Es können jedoch auch Kohlenoxid- oder wasserstoffreiche Gemische mit einem Kohlenmonoxid zu einem Wasserstoff-Anteil von 30:70 bis 70:30 (in Molen) eingesetzt werden.

Die beanspruchte Arbeitsweise ermöglicht es, ungesättigte, unter Normalbedingungen gasförmige Kohlenwasserstoffe aus Synthesegas mit hoher Selektivität und hoher Ausbeute herzustellen. Bevorzugte Produkte der Reaktion sind Ethylen, daneben Propylen und Butylene. Als Nebenprodukte entstehen die entsprechenden Alkane.

Die Durchführung des neuen Verfahrens gestaltet sich einfach und ist an keine Arbeitsweise gebunden. Die Katalysatoren werden im allgemeinen in Form eines Festbettes angeordnet. Sie können jedoch auch in feinverteilter Form als Wirbelschicht oder in der Flüssigphase eingesetzt werden.

Zur Umsetzung wird Synthesegas bei Temperaturen von 220 bis 400°C und bei Drücken bis zu 30 bar über den Katalysator geleitet. Das den Reaktionsraum verlassende Gasgemisch wird zweckmässigerweise nach Entfernung der ungesättigten Kohlenwasserstoffe nach an sich bekannten Verfahren, z.B. durch Tieftemperatur-Zerlegung oder durch adsoptive Trennung, dem Reaktor ganz oder teilweise wieder zugeführt.

Die Erfindung wird durch die nachfolgenden Beispiele näher erläutert, sie ist jedoch nicht auf die Ausführungsform dieser Beispiele beschränkt.

Beispiele

Alle Versuche werden in einem Rohrreaktor von 1,50 m Länge und 20 mm lichter Weite, in dem der durch Siliziumcarbid extern verdünnte Katalysator in einer 96 cm hohen Schicht eingefüllt ist, durchgeführt. Nachdem der Reaktor mittels einer elektronischen Heizung auf die Reaktionstemperatur gebracht ist, werden die Einsatzstoffe über den Katalysator geleitet. Die in den Versuchen eingesetzten Katalysatoren haben folgende Zusammensetzung:

100 Gew.-teile Eisen
80 Gew.-teile Vanadium
7 Gew.-teile MgO
4 Gew.-teile $K_2O$

| Gesamtlaufzeit | Reaktionsbedingungen | | | Syntheseergebnisse | | | | |
|---|---|---|---|---|---|---|---|---|
| | Temperatur | Druck | V/Vh | $U_{(CO+H_2)}$ | $U_{CO}$ | $A_{C_2-C_4}$-Olefine | $S^*_{CO+H_2}$ | $S^*_{CO}$ |
| (h) | (°C) | (bar) | ($h^{-1}$) | (%) | (%) | (g/Nm³) | (%) | (%) |
| a) Katalysator unter Verwendung von schwefelhaltigem MgO hergestellt (MgO mit 0,5% $SO_4 \triangleq$ 40 ppm bezogen auf die gesamte Katalysatormasse) | | | | | | | | |
| 383 | 330 | 10,1 | 878 | 85 | 96,1 | 58,7 | 33,1 | 29,3 |
| 407 | 330 | 10,1 | 941 | 83,3 | 97,4 | 61,4 | 35,4 | 30,2 |
| 431 | 340 | 10,1 | 887 | 84,3 | 97,4 | 67,3 | 38,3 | 33,1 |
| 469 | 340 | 10,1 | 876 | 83,9 | 95,6 | 62,5 | 35,8 | 31,3 |
| 493 | 341 | 10,1 | 981 | 84,4 | 94,6 | 58,6 | 33,3 | 29,7 |
| 517 | 341 | 10,1 | 963 | 84,9 | 97,3 | 62,8 | 35,5 | 31,0 |
| 541 | 350 | 10,1 | 953 | 83,8 | 96,7 | 67,0 | 38,3 | 33,2 |
| 565 | 350 | 10,1 | 926 | 82,5 | 96,4 | 65,9 | 38,3 | 32,8 |
| 589 | 350 | 10,1 | 891 | 80,7 | 96,0 | 71,7 | 42,7 | 35,8 |
| 613 | 351 | 10,1 | 1954 | 78,1 | 94,7 | 64,9 | 39,9 | 32,9 |
| 637 | 350 | 10,1 | 1846 | 79,8 | 93,1 | 60,0 | 36,1 | 30,9 |
| 661 | 350 | 10,1 | 1747 | 78,9 | 92,4 | 59,2 | 36,0 | 30,7 |
| 685 | 350 | 10,1 | 1855 | 78,0 | 93,7 | 61,8 | 38,0 | 31,6 |
| 709 | 349 | 10,1 | 820 | 81,1 | 94,8 | 64,6 | 38,2 | 32,6 |
| 733 | 350 | 10,1 | 443 | 84,2 | 97,3 | 64,1 | 36,5 | 31,6 |
| b) Katalysator unter Verwendung von analytisch reinem MgO hergestellt | | | | | | | | |
| 251 | 309 | 10 | 1017 | 85,3 | 95,3 | 39,3 | 22,1 | 19,7 |
| 467 | 350 | | 1188 | 89,5 | 96,7 | 47,5 | 26,2 | 23,5 |

| Gesamtlaufzeit | Reaktionsbedingungen | | | Syntheseergebnisse | | | | |
|---|---|---|---|---|---|---|---|---|
| | Temperatur | Druck | V/Vh | $U_{(CO+H_2)}$ | $U_{CO}$ | $A_{C_2-C_4}$-Olefine | $S^*_{CO+H_2}$ | $S^*_{CO}$ |
| (h) | (°C) | (bar) | ($h^{-1}$) | (%) | (%) | (g/Nm³) | (%) | (%) |
| c) Katalysator unter Verwendung eines Gemisches aus analytisch reinem MgO und $MgSO_4$ hergestellt; S-Konzentration: 40 ppm bezogen auf die gesamte Katalysatormasse | | | | | | | | |
| 599 | 330 | 10 | 945 | 84,0 | 97,1 | 58,9 | 33,7 | 29,1 |
| 623 | 343 | 10 | 918 | 83,4 | 96,4 | 59,8 | 34,8 | 29,8 |
| 647 | 338 | 10 | 927 | 86,2 | 96,6 | 62,7 | 34,9 | 31,2 |
| 671 | 340 | 10 | 918 | 84,5 | 96,1 | 59,5 | 33,8 | 29,7 |
| 719 | 340 | 10 | 927 | 83,9 | 95,8 | 59,3 | 33,9 | 29,7 |
| 791 | 349 | 10 | 999 | 83,4 | 94,8 | 62,6 | 36,0 | 31,7 |
| 839 | 350 | 10 | 999 | 83,3 | 95,5 | 66,5 | 38,3 | 33,3 |
| 863 | 350 | 10 | 927 | 83,5 | 95,4 | 55,9 | 32,1 | 28,1 |

*Schwefel

*Unter der Olefin-Selektivität S verstehen wir den prozentualen Anteil gasförmige Olefine (Ausbeute $A_{C_2^--C_4^-}$) an der bei dem entsprechenden Synthesegas- bzw. Kohlenmonoxid-Umsatz maximal erreichbaren Kohlenwasserstoffausbeute

$$S = \frac{A_{(C_2^--C_4^-)} \cdot 100}{208{,}5 \cdot U_{(CO+H_2)} \text{ bzw. } U_{CO}}$$

## Patentansprüche

1. Verfahren zur Herstellung ungesättigter niedermolekularer Kohlenwasserstoffe durch Hydrierung von Kohlenoxiden in Gegenwart von Katalysatoren, die aus 100 Gewichtsteilen Eisen, 50 bis 100 Gewichtsteilen Vanadium, bis zu 10 Gewichtsteile Magnesiumoxid und 3 bis 5 Gewichtsteile Kaliumoxid bestehen, dadurch gekennzeichnet, dass sie ausserdem 10 bis 150 ppm Schwefel (bezogen auf die gesamte Katalysatormasse) enthalten.

2. Verfahren nach Anspruch 1, dadurch gekennzeichnet, dass der Schwefel in Form von schwefelhaltigem Magnesiumoxid im Katalysator enthalten ist.

3. Verfahren nach Anspruch 1 und 2, dadurch gekennzeichnet, dass der Schwefel in Form sulfathaltigem Magnesiumoxid im Katalysator enthalten ist.

## Claims

1. A process for the preparation of unsaturated low molecular weight hydrocarbons by hydrogenating carbon oxides in the presence of catalysts, which contain 100 parts by weight of iron, 50 to 100 parts by weight of vanadium, up to 10 parts by weight of magnesium oxide and 3 to 5 parts by weight of potassium oxide, characterised in that they also contain 10 to 150 ppm sulphur (relative to the total catalyst mass).

2. A process according to claim 1 characterised in that the sulphur in the catalyst is contained in the form of sulphur-containing magnesium oxide.

3. A process according to claims 1 and 2 characterised in that the sulphur in the catalyst is contained in the form of a sulphate-containing magnesium oxide.

## Revendications

1. Procédé de préparation d'hydrocarbures insaturés à bas poids moléculaire par hydrogénation d'oxydes du carbone en présence de catalyseurs consistant en 100 parties en poids de fer, 50 à 100 parties en poids de vanadium, jusqu'à 10 parties en poids de magnésie et 3 à 5 parties en poids d'oxyde de potassium, caractérisé en ce que ces catalyseurs contiennent en outre de 10 à 150 ppm de soufre (par rapport à la masse totale du catalyseur).

2. Procédé selon la revendication 1, caractérisé en ce que le soufre est contenu dans le catalyseur sous la forme de magnésie contenant du soufre.

3. Procédé selon les revendications 1 et 2, caractérisé en ce que le soufre est contenu dans le catalyseur sous la forme de magnésie contenant un sulfate.